# EUROPEAN PATENT APPLICATION

(11) **EP 4 534 672 A1**
(43) Date of publication of application: **09.04.2025**
(21) Application number: 23816163.2
(22) Date of filing: 02.06.2023
(51) Int. Cl.: C12N 15/13, A61K 39/395, A61P 1/16, A61P 3/04, A61P 3/10, C07K 16/28, C12N 15/63, C12P 21/08

(54) **ANTIBODY THAT BINDS TO CANNABINOID TYPE 1 RECEPTORS**

(30) Priority: 03.06.2022 JP 2022090732
(71) Applicant: Shionogi & Co., Ltd, Osaka-shi, Osaka 541-0045 (JP)
(72) Inventor: YOSHIKAWA Mai, Osaka-shi, Osaka 541-0045 (JP); TAKAHASHI Tatsuya, Osaka-shi, Osaka 541-0045 (JP); ONODA Junji, Osaka-shi, Osaka 541-0045 (JP)
(74) Representative: Mewburn Ellis LLP
(86) International application number: PCT/JP2023/020578
(87) International publication number: WO 2023/234406

(57) **Abstract**

An object of the present invention is to provide a novel antibody or an antibody fragment thereof that binds to a cannabinoid type 1 (CB1) receptor. Disclosed is a CB1 receptor antibody or an antibody fragment thereof having six specific CDRs (CDR1 to CDR3 of the heavy chain and CDR1 to CDR3 of the light chain) or a specific heavy chain variable region/light chain variable region. The antibody and the like can be used for the treatment or prevention of obesity, diabetes, nonalcoholic steatohepatitis, and the like.

## Description

### [TECHNICAL FIELD]

The present invention relates to a novel antibody or an antibody fragment thereof that binds to cannabinoid type 1 (CB1) receptor. More specifically, the present invention relates to an antibody or an antibody fragment thereof that specifically binds to a CB1 receptor. Even more specifically, the present invention relates to an antibody or an antibody fragment thereof that selectively inhibits a CB1 receptor, a pharmaceutical composition comprising the same, or a kit for detecting a CB1 receptor.

### [BACKGROUND ART]

A CB1 receptor is a type of the G protein-coupled receptor (GPCR) family expressed mainly in the central nervous system, liver, kidney, lung, and adipose tissue, and mediates reactions to endogenous cannabinoids and Δ9-tetrahydrocannabinol that is a plant phytocannabinoid. When signaling by a CB1 receptor is activated by an endogenous cannabinoid or the like, interaction with Gi/O protein causes inhibition of adenyl cyclase, activation of mitogen-activated protein kinase (MAPK), inhibition of voltage-dependent Ca ion channels, influence on NO signaling, and the like (Non-Patent Document 1).

A CB1 receptor is involved in many diseases such as obesity, diabetes, pain, neurodegenerative diseases, fibrosis, liver disease, and cardiovascular disease. In fact, it has been shown that an antagonist therapeutic agent for CB1 receptor such as rimonabant has an anti-obesity effect and a metabolic improvement action such as appetite reduction and neutral fat value reduction. On the other hand, it has been revealed that a small molecule antagonist such as rimonabant causes central side effects such as mental effects, depression, and suicidal desires (Non-patent Document 2).

It has been revealed that a small molecule CB1 receptor antagonist with low migration to the brain which minimizes the risk of central side effects exhibits an improving effect on insulin resistance, anti-dyslipidemia, anti-hyperuricemia and the like in mice (Non-patent Document 3), and it can be said that a peripheral CB1 receptor inhibitor with low migration to the brain can act equally to a systemic antagonist. Thus, a CB1 receptor antagonist or a CB1 receptor agonist without central side effects is useful drug that acts broadly in the periphery.

Patent Document 1 discloses an antibody against a CB1 receptor as an example of a compound capable of modulating a CB1 receptor signal.

Patent Document 2 discloses antibodies that bind to an epitope in the EC2 domain of the human CB1 receptor. Examples of evaluating the in vitro antagonist activity and neutralizing activity of the antibodies are described, but no in vivo evaluation is described.

Patent Documents 3 and 4 disclose antibodies that are inverse agonists of a CB1 receptor, and describe in vitro and in vivo evaluation of the CB1 antibodies and the like. On the other hand, Patent Document 3 describes in [0361] to [0363] and Patent Document 4 describes in [0367] to [0369] that administration of P1C4-h2-IgG4 to primates shows a decrease in triglyceride levels and other cardiovascular risk factors and improvement in insulin sensitivity, but no specific data is disclosed.

Patent Document 5 discloses an antibody that binds to a CB1 receptor. Examples for evaluating in vitro antagonist activity and neutralizing activity are described, but no in vivo evaluation is described.

That is, in any of the prior art documents, the CDR sequence, the light chain variable region, the heavy chain variable region, and the like of the antibody of the present invention are not disclosed at all.

### [PRIOR ART REFERENCES]

### [Patent Documents]

[Patent Document 1] WO2005/023232
[Patent Document 2] WO2014/210205
[Patent Document 3] WO2015/148984
[Patent Document 4] WO2017/058771
[Patent Document 5] WO2019/211665

### [Non-patent Documents]

[Non-patent Document 1] Drug Metab Rev. 2018 Feb; 50(1): 3-13.
[Non-patent Document 2] Psychopharmacology volume 205, pages 171-174 (2009)
[Non-patent Document 3] Medicina (Kaunas). 2020 Oct 29; 56(11): 573.

### [SUMMARY OF THE INVENTION]

### [PROBLEMS TO BE SOLVED BY THE INVENTION]

An object of the present invention is to provide a novel antibody or an antibody fragment thereof that binds to a CB1 receptor. A further object of the present invention is to provide a novel CB1 receptor antibody or an antibody fragment thereof that can be used as a therapeutic agent for obesity, diabetes, and the like.

The present inventors have conducted diligent studies and found an antibody or an antibody fragment thereof that specifically binds to one or more extracellular epitopes on a CB1 receptor and modulates CB1 receptor signaling. Furthermore, it has been found that the antibody of the present invention has an improvement in small intestine transport capacity or an anti-obesity effect, and is useful for the treatment of obesity, diabetes, nonalcoholic steatohepatitis (NASH), and the like.

That is, the present invention relates to the following.
(1-1) An antibody or an antibody fragment thereof that binds to a human cannabinoid type 1 (CB1) receptor, comprising:
   a light chain variable region including
   a CDR1 having the amino acid sequence of SEQ ID NO: 44: Xaa1-A-S-S-S-V-S-Xaa2-Xaa3-Y-L-H (wherein Xaa1 is arginine or lysine, Xaa2 is threonine or serine, and
   Xaa3 is arginine or serine) in which one or two amino acids are optionally deleted, substituted, inserted and/or added,
   a CDR2 having the amino acid sequence of SEQ ID NO: 45: Xaa4-T-S-N-Xaa5-A-S (wherein Xaa4 is glycine or serine, and Xaa5 is isoleucine or leucine) in which one or two amino acids are optionally deleted, substituted, inserted and/or added, and
   a CDR3 having the amino acid sequence of SEQ ID NO: 46: Q-Q-Y-Xaa6-Xaa7-F-P-L-T (wherein Xaa6 is threonine or serine, and Xaa7 is alanine or glycine) in which one or two amino acids are optionally deleted, substituted, inserted and/or added, and
   a heavy chain variable region including
   a CDR1 having the amino acid sequence of SEQ ID NO: 24 in which one or two amino acids are optionally deleted, substituted, inserted and/or added,
   a CDR2 having the amino acid sequence of SEQ ID NO: 47: Q-I-Y-P-G-D-Xaa8-D-N-Xaa9-Y-N-Xaa10-K-F-K-G (wherein Xaa8 is glycine or alanine, Xaa9 is lysine or arginine, and Xaa10 is glycine or alanine) in which one or two amino acids are optionally deleted, substituted, inserted and/or added, and
   a CDR3 having the amino acid sequence of SEQ ID NO: 26 in which one or two amino acids are optionally deleted, substituted, inserted and/or added.
   (that is, the heavy chain variable region and the light chain variable region including a CDR of mIML1-2, m20C3, hIML1-2, hIML1-2-OP1 or hIML1-2-2 of Example or variants thereof)
(1-2) An antibody or an antibody fragment thereof that binds to a human cannabinoid type 1 (CB1) receptor, comprising:
   a1) a light chain variable region having
      a CDR1 having the amino acid sequence of SEQ ID NO: 44 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   a2) a heavy chain variable region having
      a CDR1 having the amino acid sequence of SEQ ID NO: 24 in which one or two amino acids are optionally deleted, substituted, inserted and/or added,
      a CDR2 having the amino acid sequence of SEQ ID NO: 27 in which one or two amino acids are optionally deleted, substituted, inserted and/or added, and
      a CDR3 having the amino acid sequence of SEQ ID NO: 26 in which one or two amino acids are optionally deleted, substituted, inserted and/or added;
   b1) a light chain variable region having
      a CDR1 having the amino acid sequence of SEQ ID NO: 44 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 21 in which one or two amino acids are optionally deleted, substituted, inserted and/or added, and
   b2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 having the amino acid sequence of SEQ ID NO: 26 in which one or two amino acids are optionally deleted, substituted, inserted and/or added;
   c1) a light chain variable region including
      a CDR1 having the amino acid sequence of SEQ ID NO: 44 in which one or two amino acids are optionally deleted, substituted, inserted and/or added,
      a CDR2 having the amino acid sequence of SEQ ID NO: 20 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   c2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 havingthe amino acid sequence of SEQ ID NO: 26 in which one or two amino acids are optionally deleted, substituted, inserted and/or added;
   d1) a light chain variable region including
      a CDR1 having the amino acid sequence of SEQ ID NO: 19 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   d2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 having the amino acid sequence of SEQ ID NO: 26 in which one or two amino acids are optionally deleted, substituted, inserted and/or added; or
   e1) a light chain variable region including
      a CDR1 having the amino acid sequence of SEQ ID NO: 44 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   e2) a heavy chain variable region having
      a CDR1 having the amino acid sequence of SEQ ID NO: 24 in which one or two amino acids are optionally deleted, substituted, inserted and/or added,
      a CDR2 having the amino acid sequence of SEQ ID NO: 25 in which one or two amino acids are optionally deleted, substituted, inserted and/or added, and
      a CDR3 having the amino acid sequence of SEQ ID NO: 26 in which one or two amino acids are optionally deleted, substituted, inserted and/or added
      (that is, the heavy chain variable region and the light chain variable region including a CDR of mIML1-2 or hIML1-2 of Example or variants thereof).
(1-3) An antibody or an antibody fragment thereof that binds to a human cannabinoid type 1 (CB1) receptor, comprising:
   a1) a light chain variable region including
      a CDR1 having the amino acid sequence of SEQ ID NO: 44 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   a2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 36 in which one or two amino acids are optionally deleted, substituted, inserted and/or added, and
      a CDR3 having the amino acid sequence of SEQ ID NO: 26 in which one or two amino acids are optionally deleted, substituted, inserted and/or added;
   b1) a light chain variable region including
      a CDR1 having the amino acid sequence of SEQ ID NO: 44 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 35 in which one or two amino acids are optionally deleted, substituted, inserted and/or added, and
   b2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 having the amino acid sequence of SEQ ID NO: 26 in which one or two amino acids are optionally deleted, substituted, inserted and/or added;
   c1) a light chain variable region including
      a CDR1 having the amino acid sequence of SEQ ID NO: 44 in which one or two amino acids are optionally deleted, substituted, inserted and/or added,
      a CDR2 having the amino acid sequence of SEQ ID NO: 34 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   c2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 having the amino acid sequence of SEQ ID NO: 26 in which one or two amino acids are optionally deleted, substituted, inserted and/or added; or
   d1) a light chain variable region including
      a CDR1 having the amino acid sequence of SEQ ID NO: 33 in which one or two amino acids are optionally deleted, substituted, inserted and/or added,
      a CDR2 having the amino acid sequence of SEQ ID NO: 45 in which one or two amino acids are optionally deleted, substituted, inserted and/or added, and
      a CDR3 having the amino acid sequence of SEQ ID NO: 46 in which one or two amino acids are optionally deleted, substituted, inserted and/or added, and
   d2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 47 in which one or two amino acids are optionally deleted, substituted, inserted and/or added, and
      a CDR3 having the amino acid sequence of SEQ ID NO: 26 in which one or two amino acids are optionally deleted, substituted, inserted and/or added
      (that is, the heavy chain variable region and the light chain variable region including a CDR of hIML1-2-OP1 of Example or variants thereof).
(2) An antibody or an antibody fragment thereof that binds to a human cannabinoid type 1 (CB1) receptor, comprising:
   a1) a light chain variable region including
      a CDR1 having the amino acid sequence of SEQ ID NO: 33 in which one or two amino acids are optionally deleted, substituted, inserted and/or added,
      a CDR2 having the amino acid sequence of SEQ ID NO: 34 in which one or two amino acids are optionally deleted, substituted, inserted and/or added, and
      a CDR3 having the amino acid sequence of SEQ ID NO: 35 in which one or two amino acids are optionally deleted, substituted, inserted and/or added, and
   a2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 36 in which one or two amino acids are optionally deleted, substituted, inserted and/or added, and
      a CDR3 having the amino acid sequence of SEQ ID NO: 26 in which one or two amino acids are optionally deleted, substituted, inserted and/or added
      (that is, the heavy chain variable region and the light chain variable region including a CDR of hIML1-2-OP1 of Example or variants thereof);
   b1) a light chain variable region including
      a CDR1 having the amino acid sequence of SEQ ID NO: 40 in which one or two amino acids are optionally deleted, substituted, inserted and/or added,
      a CDR2 having the amino acid sequence of SEQ ID NO: 20 in which one or two amino acids are optionally deleted, substituted, inserted and/or added, and
      a CDR3 having the amino acid sequence of SEQ ID NO: 23 in which one or two amino acids are optionally deleted, substituted, inserted and/or added, and
   b2) a heavy chain variable region including
      a CDR1 having the amino acid sequence of SEQ ID NO: 24 in which one or two amino acids are optionally deleted, substituted, inserted and/or added,
      a CDR2 having the amino acid sequence of SEQ ID NO: 28 in which one or two amino acids are optionally deleted, substituted, inserted and/or added, and
      a CDR3 having the amino acid sequence of SEQ ID NO: 29 in which one or two amino acids are optionally deleted, substituted, inserted and/or added
      (that is, the heavy chain variable region and the light chain variable region including a CDR of m2F8 or h2F8 of Example or variants thereof),
   c1) a light chain variable region including
      a CDR1 having the amino acid sequence of SEQ ID NO: 19 in which one or two amino acids are optionally deleted, substituted, inserted and/or added,
      a CDR2 having the amino acid sequence of SEQ ID NO: 20 in which one or two amino acids are optionally deleted, substituted, inserted and/or added, and
      a CDR3 having the amino acid sequence of SEQ ID NO: 21, and
      a heavy chain variable region including
         c2-1) a CDR1 having the amino acid sequence of SEQ ID NO: 24 in which one or two amino acids are optionally deleted, substituted, inserted and/or added,
            a CDR2 having the amino acid sequence of SEQ ID NO: 27 in which one or two amino acids are optionally deleted, substituted, inserted and/or added, and
            a CDR3 having the amino acid sequence of SEQ ID NO: 26 in which one or two amino acids are optionally deleted, substituted, inserted and/or added, or
         c2-2) a CDR1 having the amino acid sequence of SEQ ID NO: 24 in which one or two amino acids are optionally deleted, substituted, inserted and/or added,
            a CDR2 having the amino acid sequence of SEQ ID NO: 25 in which one or two amino acids are optionally deleted, substituted, inserted and/or added, and
            a CDR3 having the amino acid sequence of SEQ ID NO: 26 in which one or two amino acids are optionally deleted, substituted, inserted and/or added
            (that is, the heavy chain variable region and the light chain variable region including a CDR of m20C3, mIML1-2hIML1-2 or hIML1-2-2 of Example or variants thereof); or
         d1) a light chain variable region including
            a CDR1 having the amino acid sequence of SEQ ID NO: 22 in which one or two amino acids are optionally deleted, substituted, inserted and/or added,
            a CDR2 having the amino acid sequence of SEQ ID NO: 20 in which one or two amino acids are optionally deleted, substituted, inserted and/or added, and
            a CDR3 having the amino acid sequence of SEQ ID NO: 23 in which one or two amino acids are optionally deleted, substituted, inserted and/or added, and
            a heavy chain variable region including
         d2-1) a CDR1 having the amino acid sequence of SEQ ID NO: 24 in which one or two amino acids are optionally deleted, substituted, inserted and/or added,
            a CDR2 having the amino acid sequence of SEQ ID NO: 28 in which one or two amino acids are optionally deleted, substituted, inserted and/or added, and
            a CDR3 having the amino acid sequence of SEQ ID NO: 30 in which one or two amino acids are optionally deleted, substituted, inserted and/or added,
         d2-2) a CDR1 having the amino acid sequence of SEQ ID NO: 24 in which one or two amino acids are optionally deleted, substituted, inserted and/or added,
            a CDR2 having the amino acid sequence of SEQ ID NO: 28 in which one or two amino acids are optionally deleted, substituted, inserted and/or added, and
            a CDR3 having the amino acid sequence of SEQ ID NO: 31 in which one or two amino acids are optionally deleted, substituted, inserted and/or added, or
         d2-3) a CDR1 having the amino acid sequence of SEQ ID NO: 24 in which one or two amino acids are optionally deleted, substituted, inserted and/or added,
            a CDR2 having the amino acid sequence of SEQ ID NO: 28 in which one or two amino acids are optionally deleted, substituted, inserted and/or added, and
            a CDR3 having the amino acid sequence of SEQ ID NO: 32 in which one or two amino acids are optionally deleted, substituted, inserted and/or added
            (that is, the heavy chain variable region and the light chain variable region including a CDR of m4D7, m24C4 or m26C7 of Example or variants thereof).
(3-1) The antibody or an antibody fragment thereof according to any one of (1-1) to (1-3) and (2), comprising:
   a light chain variable region having
   the amino acid sequences of SEQ ID NOs: 13 to 15, or
   the amino acid sequences at least 95% identical to the amino acid sequences of SEQ ID NOs: 13 to 15, and
   a heavy chain variable region having
      the amino acid sequences of SEQ ID NOs: 16 to 18, and 52, or
      the amino acid sequences at least 95% identical to the amino acid sequences of SEQ ID NOs: 16 to 18, and 52.
(3-2) The antibody or an antibody fragment thereof according to any one of (1-1) to (1-3) and (2), comprising:
   a light chain variable region having
   the amino acid sequences of SEQ ID NOs: 1 to 4, or
   the amino acid sequences at least 95% identical to the amino acid sequences of SEQ ID NOs: 1 to 4, and
   a heavy chain variable region having
      the amino acid sequences of SEQ ID NOs: 7 to 12, or
      the amino acid sequences at least 95% identical to the amino acid sequences of SEQ ID NOs: 7 to 12.
(4) The antibody or an antibody fragment thereof according to any one of (1-1) to (1-3), (2), and (3-1), comprising:
   a1) a light chain variable region having the amino acid sequence of SEQ ID NO: 14, and
      a heavy chain variable region having the amino acid sequence of SEQ ID NO: 17;
   a2) a light chain variable region having the amino acid sequence of SEQ ID NO: 15, and
      a heavy chain variable region having the amino acid sequence of SEQ ID NO: 18;
   a3) a light chain variable region having the amino acid sequence of SEQ ID NO: 13, and
      a heavy chain variable region having the amino acid sequence of SEQ ID NO: 16; or
   a4) a light chain variable region having the amino acid sequence of SEQ ID NO: 13, and
      a heavy chain variable region having the amino acid sequence of SEQ ID NO: 52.
(5) The antibody or an antibody fragment thereof according to any one of (1-1) to (1-3), (2), (3-1), (3-2), and (4), further comprising a heavy chain constant region having the amino acid sequence of SEQ ID NO: 37 and a light chain constant region having the amino acid sequence of SEQ ID NO: 38.
(6) A pharmaceutical composition comprising the antibody or an antibody fragment thereof according to any one of (1-1) to (1-3), (2), (3-1), (3-2), (4), and (5).
(7) The pharmaceutical composition according to (6), which is a therapeutic and/or preventive agent for obesity, diabetes, and/or nonalcoholic steatohepatitis (NASH).
(8) A polynucleotide encoding the heavy chain variable region of the antibody according to (3-1), (3-2), or (4), and
   further optionally encoding the heavy chain constant region of the antibody according to (5).
(9) A polynucleotide encoding the light chain variable region of the antibody according to (3-1), (3-2), or (4), and
   further optionally encoding the light chain constant region of the antibody according to (5).
(10) An expression vector comprising the polynucleotide according to (8) or (9).
(11) An antibody or an antibody fragment thereof that binds to a human cannabinoid type 1 (CB1) receptor, comprising:
   a1) a light chain variable region including
      a CDR1 having the amino acid sequence of SEQ ID NO: 39: Xaa11-A-S-S-S-V-S-Xaa12-Xaa13-Y-L-H (wherein Xaa11 is threonine, isoleucine, arginine or lysine, Xaa12 is serine or threonine, and Xaa13 is serine or arginine),
      a CDR2 having the amino acid sequence of SEQ ID NO: 45: Xaa14-T-S-N-Xaa15-A-S (wherein Xaa14 is serine or glycine, and Xaa15 is leucine or isoleucine), and
      a CDR3 having the amino acid sequence of SEQ ID NO: 41: Xaa16-Q-Y-Xaa17-Xaa18-Xaa19-P-Xaa20-T (wherein Xaa16 is histidine or glutamine, Xaa17 is histidine, threonine or serine, Xaa18 is arginine, alanine or glycine, Xaa19 is serine or phenylalanine, and Xaa20 is arginine or leucine), and
   a2) a heavy chain variable region including
      a CDR1 having the amino acid sequence of SEQ ID NO: 24,
      a CDR2 having the amino acid sequence of SEQ ID NO: 42: Q-I-Y-P-G-D-Xaa21-D-Xaa22-Xaa23-Y-N-Xaa24-K-F-K-G (wherein Xaa21 is glycine or alanine, Xaa22 is threonine or asparagine, Xaa23 is asparagine, lysine or arginine, and Xaa24 is glycine or alanine), and
      a CDR3 having the amino acid sequence of SEQ ID NO: 43: Xaa25-Xaa26-Xaa27-Xaa28-Xaa29-Xaa30-Xaa31-Y (wherein Xaa25 is serine or threonine, Xaa26 is histidine or glycine, Xaa27 is glycine or serine, Xaa28 is asparagine or glycine, Xaa29 is tyrosine or arginine, Xaa30 is methionine or phenylalanine, and Xaa31 is isoleucine, alanine, aspartic acid or histidine)
      (that is, the heavy chain variable region and the light chain variable region including a CDR of mIML1-2, m20C3, m2F8, m4D7, m24C4, m26C7, hIML1-2, hIML1-2-OP1, hIML1-2-2 or h2F8 of Example or a variant thereof).
(12) An antibody or an antibody fragment thereof that binds to a human cannabinoid type 1 (CB1) receptor, comprising:
   a1) a light chain variable region including
      a CDR1 having the amino acid sequence of SEQ ID NO: 5: Xaa32-A-S-S-S-V-S-Xaa33-Xaa34-Y-L-H (wherein Xaa32 is isoleucine, lysine or arginine, Xaa33 is serine or threonine, and Xaa34 is serine or arginine),
      a CDR2 having the amino acid sequence of SEQ ID NO: 45, and
      a CDR3 having the amino acid sequence of SEQ ID NO: 41, and
   a2) a heavy chain variable region including
      a CDR1 having the amino acid sequence of SEQ ID NO: 24,
      a CDR2 having the amino acid sequence of SEQ ID NO: 42, and
      a CDR3 having the amino acid sequence of SEQ ID NO: 6: Xaa35-Xaa36-Xaa37-Xaa38-Xaa39-Xaa40-Xaa41-Y (wherein Xaa35 is serine or threonine, Xaa36 is histidine or glycine, Xaa37 is glycine or serine, Xaa38 is asparagine or glycine, Xaa39 is tyrosine or arginine, Xaa40 is methionine or phenylalanine, and Xaa41 is isoleucine or alanine)
      (that is, the heavy chain variable region and the light chain variable region including a CDR of hIML1-2, hIML1-2-OP1, hIML1-2-2 or h2F8 of Example or variants thereof);
   b1) a light chain variable region including
      a CDR1 having the amino acid sequence of SEQ ID NO: 48: Xaa42-A-S-S-S-V-S-S-S-Y-L-H (wherein Xaa42 is threonine, isoleucine or arginine),
      a CDR2 having the amino acid sequence of SEQ ID NO: 20, and
      a CDR3 having the amino acid sequence of SEQ ID NO: 49: Xaa43-Q-Y-Xaa44-Xaa45-Xaa46-P-Xaa47-T (wherein Xaa43 is histidine or glutamine, Xaa44 is histidine or serine, Xaa45 is arginine or glycine, Xaa46 is serine or phenylalanine, and Xaa47 is arginine or leucine), and
   b2) a heavy chain variable region including
      a CDR1 having the amino acid sequence of SEQ ID NO: 24,
      a CDR2 having the amino acid sequence of SEQ ID NO: 50: Q-I-Y-P-G-D-Xaa48-D-Xaa49-Xaa50-Y-N-Xaa51-K-F-K-G (Xaa48 is glycine or alanine, Xaa49 is threonine or asparagine, Xaa50 is asparagine or lysine, and Xaa51 is glycine or alanine), and
      a CDR3 having the amino acid sequence of SEQ ID NO: 43
      (that is, the heavy chain variable region and the light chain variable region including a CDR of mIML1-2, m20C3, m2F8, m4D7, m24C4, or m26C7 of Example or variants thereof).
(13) A polynucleotide encoding the antibody or an antibody fragment according to any of (1-1) to (1-3), (2), (3-1), (3-2), (4), (5), (11), and (12).
(14) The antibody or an antibody fragment according to any one of (1-1) to (1-3), (2), (3-1), (3-2), (4), (5), (11), and (12), wherein the antibody fragment is Fab, Fab', F(ab')2, scFv, dsFv, or a Diabody.
(15) A method for preventing or treating a CB1 receptor-related disease, comprising administering the antibody or an antibody fragment thereof according to any one of (1-1) to (1-3), (2), (3-1), (3-2), (4), (5), (11), and (12).
(16) The antibody or an antibody fragment thereof according to any one of (1-1) to (1-3), (2), (3-1), (3-2), (4), (5), (11), and (12) for producing a therapeutic or prophylactic agent for a CB1 receptor-related disease.
(17) The antibody or an antibody fragment thereof according to any one of (1-1) to (1-3), (2), (3-1), (3-2), (4), (5), (11), and (12) for the treatment or prevention of a CB1 receptor-related disease.
(18) The method according to (15), or the antibody or an antibody fragment thereof according to (16) or (17), wherein the CB1 receptor-related disease is obesity and/or diabetes.
(19) A CB1 receptor-related disease diagnosis kit comprising the antibody or an antibody fragment thereof according to any one of (1-1) to (1-3), (2), (3-1), (3-2), (4), (5), (11), and (12).
(20) The antibody or an antibody fragment thereof according to any one of (1-1) to (1-3), (2), (3-1), (3-2), (4), (5), (11) and (12), which is a humanized antibody or an antibody fragment thereof.
(21) The antibody or an antibody fragment thereof according to any one of (1-1) to (1-3), (2), (3-1), (3-2), (4), (5), (11) and (12), which is a fully human antibody.
(22) A pharmaceutical composition comprising the antibody or an antibody fragment thereof according to any one of (1-1) to (1-3), (2), (3-1), (3-2), (4), (5), (11), and (12), for use in modulating CB1 receptor signaling activity.
(23) A pharmaceutical composition comprising the antibody or an antibody fragment thereof according to any one of (1-1) to (1-3), (2), (3-1), (3-2), (4), (5), (11), and (12), which is an antagonist or inverse agonist for CB1 receptor signaling activity.

### [EFFECTS OF THE INVENTION]

An antibody or an antibody fragment of the present invention specifically binds to a CB1 receptor and thus can be used for detecting a CB1 receptor in a biological sample. Furthermore, since an antibody or an antibody fragment of the present invention has the activity of modulating CB1 receptor signaling, a pharmaceutical composition comprising an antibody or an antibody fragment of the present invention is very useful as a medicament, particularly as a medicament for the treatment or prevention of a CB1 receptor-related disease.

### [BRIEF DESCRIPTION OF DRAWINGS]

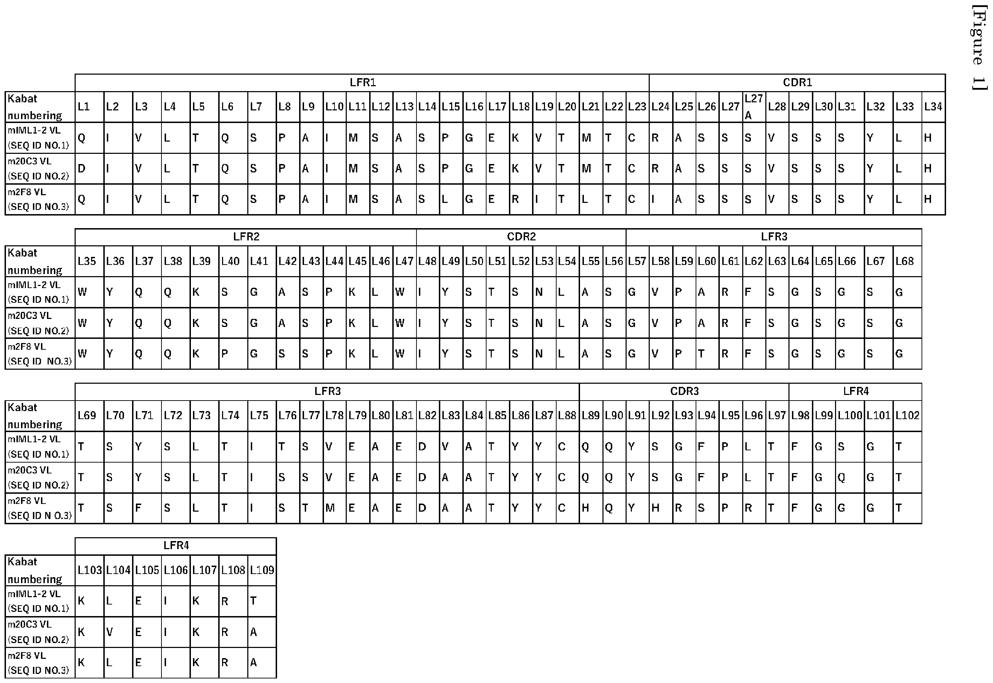

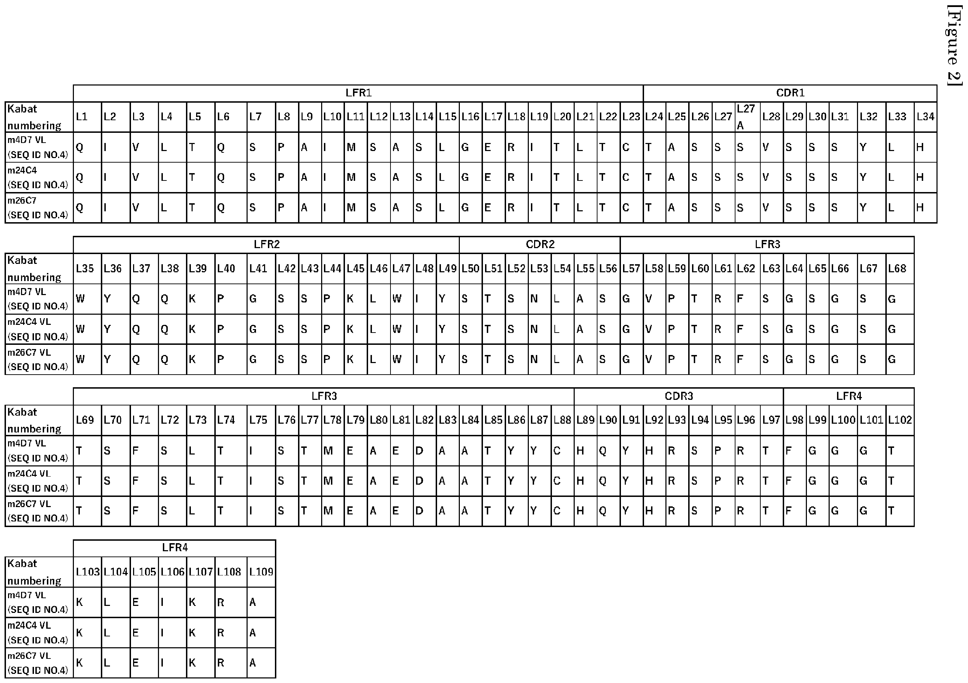

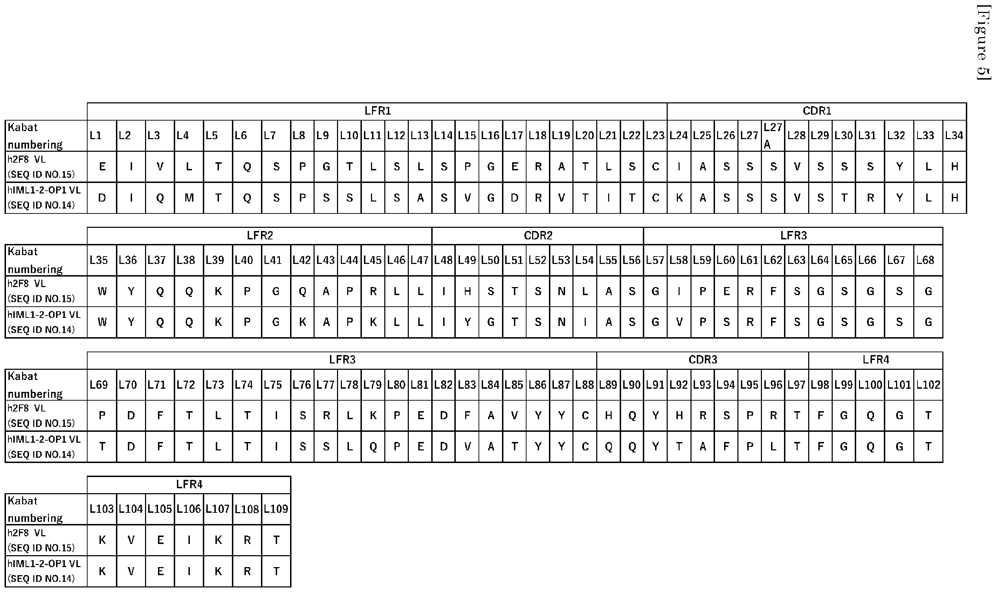

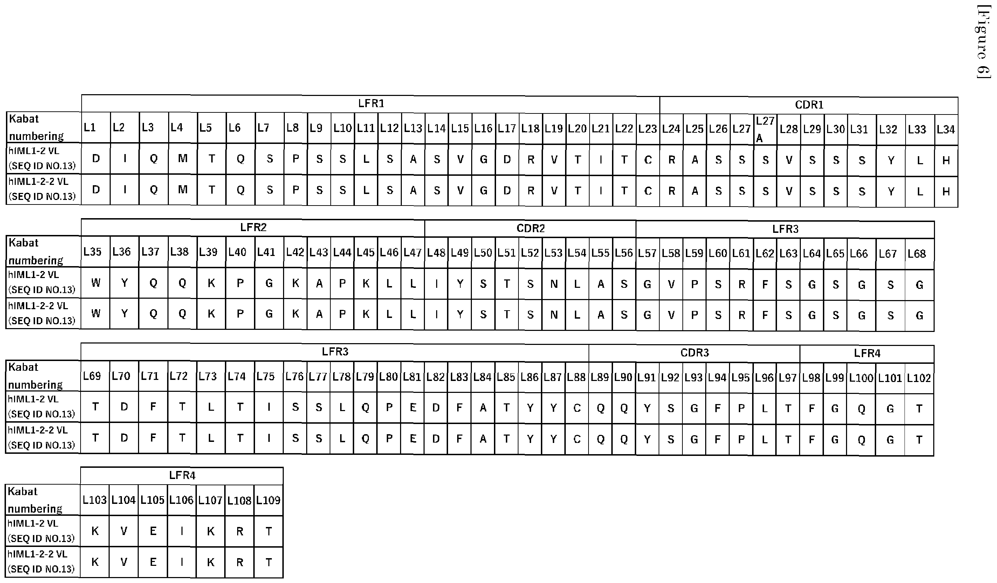

Figure 1 shows the Kabat numbering for a light chain variable region of mIML1-2 (SEQ ID NO: 1), a light chain variable region of m20C3 (SEQ ID NO: 2), and a light chain variable region of m2F8 (SEQ ID NO: 3) (LFR1 to LFR4 each mean light chain framework regions 1 to 4, CDR1 to CDR3 each mean complementarity determining regions 1 to 3, and they are also synonymous in Figures 2, 5, and 6.).
Figure 2 shows the Kabat numbering for light chain variable regions of m4D7, m24C4, and m26C7 (SEQ ID NO: 4).

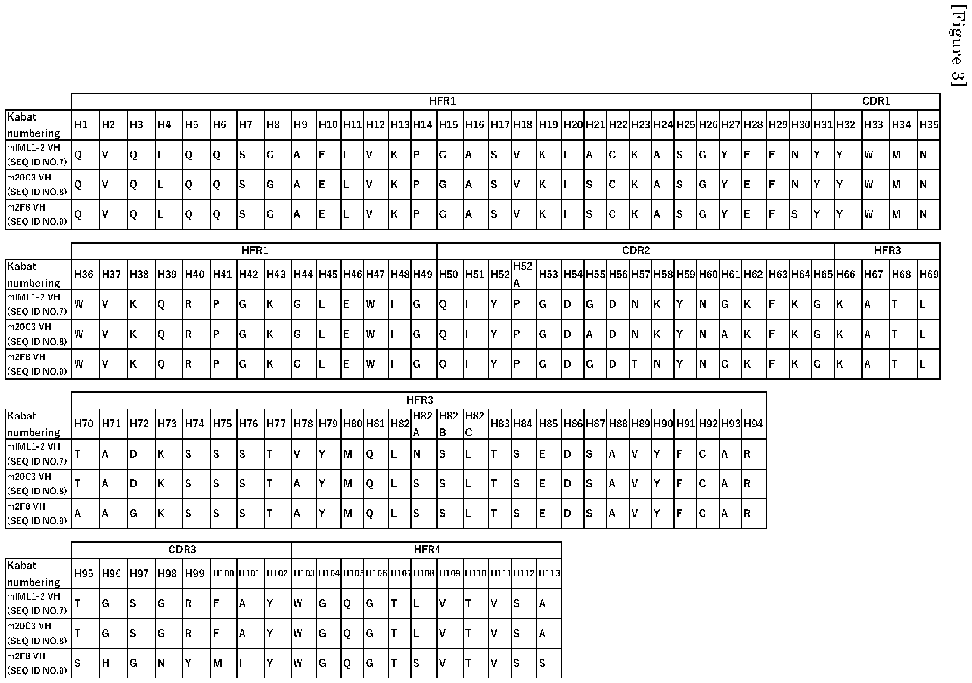

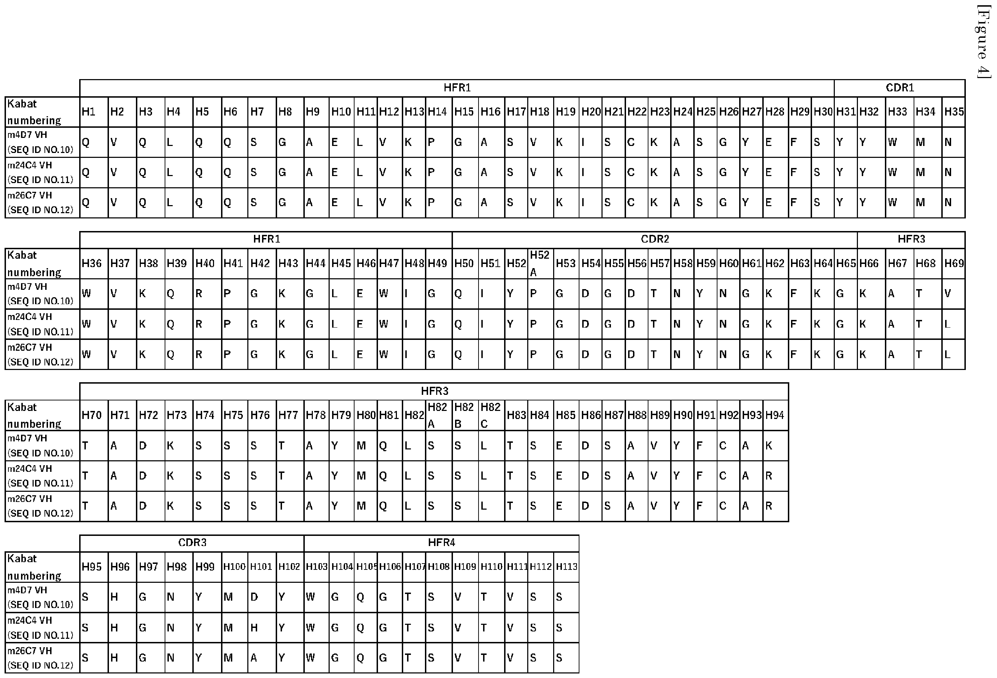

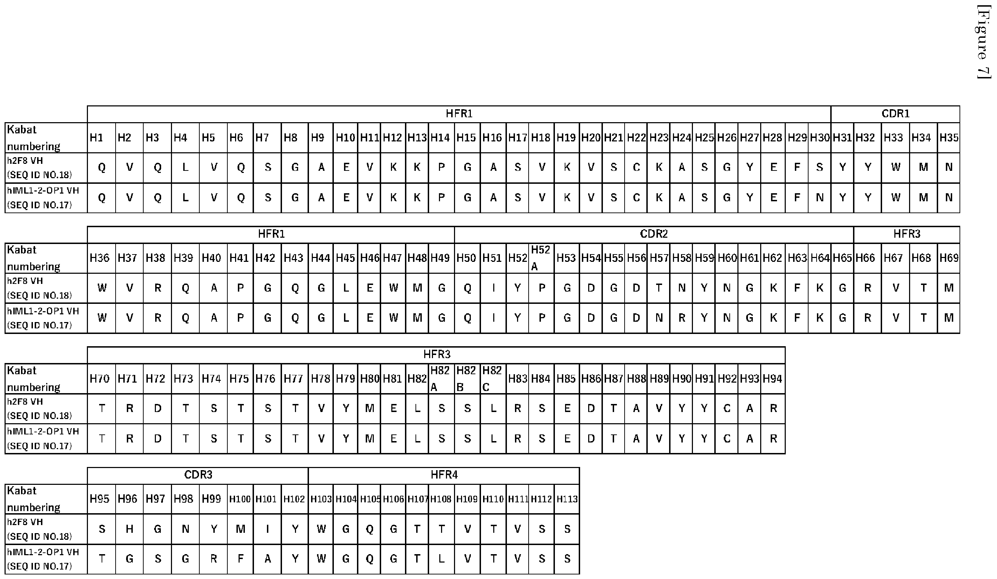

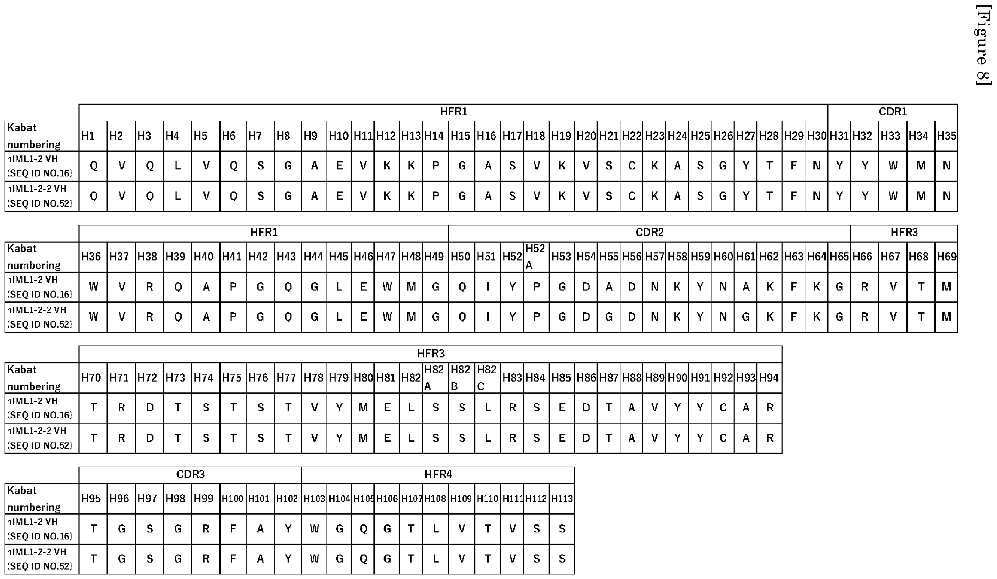

Figure 3 shows the Kabat numbering for a heavy chain variable region of mIML1-2 (SEQ ID NO: 7), a heavy chain variable region of m20C3 (SEQ ID NO: 8), and a heavy chain variable region of m2F8 (SEQ ID NO: 9) (HFR1 to HFR4 each mean heavy chain framework regions 1 to 4, CDR1 to CDR3 each mean complementarity determining regions 1 to 3, and they are also synonymous in Figures 4, 7, and 8.).
Figure 4 shows the Kabat numbering for a heavy chain variable region of m4D7 (SEQ ID NO: 10), a heavy chain variable region of m24C4 (SEQ ID NO: 11), and a heavy chain variable region of m26C7 (SEQ ID NO: 12).
Figure 5 shows the Kabat numbering for a light chain variable region of h2F8 (SEQ ID NO: 15) and a light chain variable region of hIML1-2-OP1 (SEQ ID NO: 14).
Figure 6 shows the Kabat numbering for a light chain variable region of hIML1-2 (SEQ ID NO: 13) and a light chain variable region of hIML1-2-2 (SEQ ID NO: 13).
Figure 7 shows the Kabat numbering for a heavy chain variable region of h2F8 (SEQ ID NO: 18) and a heavy chain variable region of hIML1-2-OP1 (SEQ ID NO: 17).
Figure 8 shows the Kabat numbering for a heavy chain variable region of hIML1-2 (SEQ ID NO: 16) and a heavy chain variable region of hIML1-2-2 (SEQ ID NO: 52).
Figure 9 shows evaluation of in vivo antagonistic action (improvement in small intestine transport capacity) of anti-human CB1 receptor antibody.
Figure 10 shows evaluation of anti-obesity effect (body weight change amount) of anti-human CB1 receptor antibody.
Figure 11 shows evaluation of anti-obesity effect (amount of triglyceride in the liver) of anti-human CB1 receptor antibody.

The term used in the present description is used in the meaning usually used in the art, unless specifically mentioned.

Antibody production techniques known in the art can be used in the present invention. Examples of the techniques include a method described in Immunochemistry in Practice (Blackwell Scientific Publiations).

Genetically engineered techniques known in the art can also be used. Examples of the techniques include methods described in Molecular Cloning, A Laboratory Manual, Forth Edition, Cold Spring Harbor Laboratory Press (2012), and Current Protocols Essential Laboratory Techniques, Current Protocols (2012).

The human cannabinoid (CB1) receptor is a protein consisting of amino acids encoded by the gene CNR1 (UniProtKB/Swiss-Prot: P21554: SEQ ID NO: 51). The extramembrane domains of human CB1 receptor correspond to the N-terminal region consisting of amino acids positions 1-116, the loop1 region consisting of amino acids positions 176-187 (EC loop 1), the loop2 region consisting of amino acids positions 256-273 (EC loop 2), and the loop3 region consisting of amino acids positions 366-377 (EC loop 3).

In the present specification, the terms "CB1 receptor" and "CB1" both mean a cannabinoid type 1 receptor.

An antibody or an antibody fragment of the present invention is an antibody or an antibody fragment thereof having a CDR or a heavy chain variable region/light chain variable region as described in the present description. The antibody or an antibody fragment may be from any class or subclass of immunoglobulin molecule (e.g., IgG, IgE, IgM, IgD, or IgA). The antibody or an antibody fragment may also be obtained from any species, such as mouse, rat, shark, rabbit, pig, hamster, camel, llama, goat, or human. The antibody or an antibody fragment is preferably a humanized antibody or an antibody fragment of a humanized antibody.

In the present invention, the "antibody fragment of an antibody" means a portion of the antibody of the present invention and a fragment that specifically binds to CB1 receptor and has an activity of modulating CB1 receptor signaling in the same manner as the antibody.

Specifically, examples of the antibody fragment include Fab (fragment of antigen binding), Fab', F(ab')2, a single chain antibody (single chain Fv; hereinafter referred to as scFv), a disulfide stabilized antibody (disulfide stabilized Fv; hereinafter referred to as dsFv), a dimerized V-region fragment (hereinafter referred to as Diabody), a peptide comprising a CDR, and the like, which specifically binds to the human CB1 receptor (Expert Opinion on Therapeutic Patents, vol. 6, No. 5, pp. 441-456, 1996).

Fab is an antibody fragment having an antigen-binding activity of about 50,000 molecular weight, composed of about half of the N-terminal end side of the H-chain and the entire L-chain obtained by degrading the peptide portion of the upper part of the two disulfide bonds (S-S bonds) crosslinking the two H-chains in the hinge region of IgG with an enzyme papain. Fab used in the present invention can be obtained by treating the antibody of the present invention with papain. Fab can also be produced by inserting DNA encoding Fab of the antibody of the present invention into a cell expression vector, and introducing the obtained vector into a cell to express Fab.

Fab' is an antibody fragment having an antigen-binding activity of about 50,000 molecular weight, obtained by cleaving S-S bonds in the hinge of F(ab')2. Fab' used in the present invention can be obtained by treating F(ab')2 of the antibody of the present invention with a reducing agent dithiothreitol. Fab' can also be produced by inserting DNA encoding Fab' of the antibody of the present invention into a cell expression vector, and introducing the obtained vector into E. coli, yeast, or an animal cell to express Fab'.

F(ab')2 is an antibody fragment having an antigen-binding activity of about 100,000 molecular weight, composed of two Fab' regions bonded at the hinge portion obtained by degrading the lower part of the two S-S bonds in the hinge region of IgG with an enzyme pepsin. F(ab')2 used in the present invention can be obtained by treating the antibody of the present invention with pepsin. F(ab')2 can also be produced by inserting DNA encoding F(ab')2 of the antibody of the present invention into a cell expression vector, and introducing the obtained vector into E. coli, yeast, or an animal cell to express F(ab')2.

scFv is a VH-P-VL or VL-P-VH polypeptide in which one VH and one VL are linked with an appropriate peptide linker (hereinafter referred to as P), and is an antibody fragment having antigen activity. The VH and VL contained in scFv used in the present invention may be those of the antibody of the present invention. scFv used in the present invention can also be produced by constructing a scFv expression vector using cDNA encoding the VH and VL of the antibody of the present invention and introducing the obtained vector into E. coli, yeast, or an animal cell to express scFv.

dsFv refers to an antibody fragment obtained by bonding polypeptides in which 1 amino acid residue in VH and VL is substituted with a cysteine residue, respectively, via an S-S bond. The amino acid residues to be substituted with cysteine residues can be selected based on stereostructural predictions of the antibody according to the method shown by Reiter et al. (Protein Engineering, 7, 697 (1994)). The VH or VL contained in the dsFv used in the present invention may be those of the antibody of the present invention. dsFv used in the present invention can also be produced by constructing a dsFv expression vector by inserting cDNA encoding the VH and VL of the antibody of the present invention into an appropriate expression vector and introducing the obtained vector into E. coli, yeast, or an animal cell to express dsFv.

The Diabody is an antibody fragment in which scFvs having the same or different antigen-binding specificity form a dimer, and is an antibody fragment having divalent antigen-binding activity for the same antigen or two different specific antigen-binding activities for different antigens. For example, a divalent Diabody that specifically reacts to the antibody of the present invention can be produced by using cDNA encoding the VH and VL of the antibody of the present invention to construct a DNA encoding scFv having a peptide linker of 3 to 10 residues, inserting the DNA into a cell expression vector, introducing the obtained expression vector into E. coli, yeast, or an animal cell to express the Diabody.

A peptide comprising a CDR is composed of at least one or more regions of a CDR of VH or VL. The plurality of CDRs can be bound directly or via an appropriate peptide linker. The peptide comprising a CDR used in the present invention can be produced by constructing a CDR-encoding DNA using cDNA encoding the VH and VL of the antibody of the present invention, inserting the DNA into an animal cell expression vector, and introducing the obtained vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

The antibody or an antibody fragment of the present invention is characterized in that it specifically binds to a CB1 receptor.

The specific binding can be characterized by an equilibrium dissociation constant of at least about 1 × 10⁻⁶ M or less (for example, the smaller KD represents the closer binding). The Kd value is preferably 1 x 10⁻⁷ M or less, more preferably 1 x 10⁻⁸ M or less, still more preferably 1 x 10⁻⁹ M or less. Methods for determining whether two molecules specifically bind are well known in the art, and examples thereof include competitive ELISA methods, surface plasmon resonance, and the like other method. However, an isolated antibody that specifically binds to a CB1 receptor may give rise to antibodies such as CB1 receptor molecules from other species to show cross-reactivity to target antigens other than antigens. Nevertheless, furthermore, a multispecific antibody that binds to a CB1 receptor and one or more additional antigens, or a bispecific antibody that binds to two different regions of a CB1 receptor (for example, EC loop 1 and EC loop 2) is considered as an antibody that "specifically binds" to a CB1 receptor.

The antibody or an antibody fragment of the present invention is characterized by having an activity of modulating CB1 receptor signaling. The activity of modulating CB1 receptor signaling means an activity of inhibiting or antagonizing, an activity of inverse-agonizing, or an activity of enhancing or agonizing CB1 receptor signaling. The antibody or an antibody fragment of the present invention is preferably an antibody or an antibody fragment having CB1 receptor inverse agonist activity or CB1 receptor antagonist activity. The CB1 receptor inverse agonist activity refers to an activity that reduces CB1 receptor activity to less than basal levels, and the CB1 receptor antagonist activity refers to an activity that inhibits, reduces, or blocks signaling activity of a ligand of a CB1 receptor.

The measurement procedure of CB1 receptor inverse agonist activity can be determined, for example, by measuring Ca²⁺ influx by addition of a CB1 receptor antagonist (CP55940 or the like) using CB1 receptor cells expressing a human CB1 receptor, and calculating the IC50 value by setting the inhibition rate when the CB1 receptor antagonist is not added as 100% and the inhibition rate when the CB1 receptor antagonist is added and the antibody is not added as 0% (see Example 4). In addition, the cAMP assay described in Example 4, aequorin assay, or GTP-Eu assay may be used to determine whether signaling is suppressed.

In addition to the above, the antibody or an antibody fragment of the present invention has any or all of the following excellent characteristics:
a) exhibiting a strong anti-fibrotic effect,
b) having intense effect for suppressing small intestine transport,
c) having intense efficacy against anti-obesity,
d) having low immunogenicity in humans,
e) having high stability, and
f) having good kinetics and high blood stability.

The antibody of the present invention can be produced by a conventional method in the art using a CDR or a heavy chain variable region/light chain variable region described in the present description.

The antibody of the present invention also includes monoclonal, monospecific, single-chain, chimeric, humanized, fully human, synthetic, recombinant, hybrid, mutated, grafted antibodies, antibody-small molecule complexes (ADC) and bispecific antibody humanized antibodies.

The humanized antibody is useful when administered to humans for therapeutic purposes or the like, because it is less antigenic in humans. A humanized antibody is an antibody in which a complementarity determining region (CDR) of a non-human mammal antibody, such as a mouse antibody, is implanted into a framework region (FR) of a human antibody. The FR of a humanized antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized antibody form an appropriate antigen binding site (Sato, K. et al., Cancer Res. 1993, vol. 53, p. 851). The percentage of amino acids of the FR to be substituted is from 0 to 15%, preferably from 0 to 5%, of the total FR region.

A fully human antibody specific to a CB1 receptor can be produced, for example, by immunizing a transgenic mouse having a human immunoglobulin gene with a CB1 receptor antigen, obtaining lymph cells from a mouse expressing the antibody, and then using hybridoma cells in which a myeloid cell line is fused to lymphocytes.

It should be noted that the humanized antibody of the present invention uses a constant region of a human antibody. Preferred examples of the constant region of a human antibody include Cy, such as Cy1, Cy2, Cy3, or Cy4, as the heavy chain, and Cκ and Cλ, as the light chain. The C-region of the human antibody may be modified to improve stability of the antibody or its production. The human antibody used in producing the humanized antibody may be any isotype of human antibody, such as IgG, IgM, IgA, IgE or IgD, and, in the present invention, IgG is preferably used, and IgG1 or IgG4 is further preferably used.

Preferably, the humanized antibody of the present invention has a light chain constant region of the amino acid sequence of SEQ ID NO: 38 and a heavy chain constant region of the amino acid sequence of SEQ ID NO: 37. However, the C-terminal lysine or the C-terminal two amino acid residues (glycine-lysine) of SEQ ID NO: 37 may or may not be present.

The humanized antibody can be made by general production methods (see, e.g., Example 4 below, WO 95/14041, WO 96/02576). Specifically, at first, a DNA sequence encoding a variable region designed to link a CDR of a mouse antibody to a FR of a human antibody is synthesized by PCR method from several oligonucleotides which are made to have moieties overlapping the terminal ends (see, WO 98/13388). The obtained DNA is linked to a DNA encoding a constant region of a human antibody, and then incorporated into an expression vector. Alternatively, a DNA encoding a variable region of an antibody may be incorporated into an expression vector comprising a DNA of a constant region of an antibody. To produce an antibody used in the present invention, the antibody gene is incorporated into an expression vector such that it is expressed under the control of an expression control region, e.g., an enhancer/promoter. The expression vector can then be used to transform a host cell and express the antibody.

Examples of the host cell of the transformant include a vertebrate cell such as a COS cell or a CHO cell, a prokaryotic cell, and a yeast. The transformant can be cultured according to a method well known to those skilled in the art, and the antibody of the present invention is produced intracellularly or extracellularly from the transformant. The medium used for the culture can be selected as appropriate depending on the adopted host cell from various types of media commonly used. When the host cell is a COS cell, examples of the medium include a medium such as RPMI-1640 medium or Dulbecco's Modified Eagle Minimum Essential Medium (DMEM) supplemented with serum components such as bovine fetal serum (FBS), as necessary. The culture temperature during culture of the transformant may be any temperature that does not significantly reduce protein synthesis capacity in the cell, and is preferably 32 to 42°C, most preferably 37°C. As necessary, the transformant can be cultured in an atmosphere containing 1 to 10% (v/v) of carbon dioxide.

Fractions comprising the antibody of the present invention produced intracellularly or extracellularly from the transformant as described above can be separated and purified by various known separation methods utilizing the physical and chemical properties or the like of the proteins. Specific examples of such methods include usual treatment with a protein precipitant, ultrafiltration, various chromatography such as molecular sieve chromatography (gel filtration), adsorption chromatography, ion exchange chromatography, affinity chromatography, or high-performance liquid chromatography (HPLC), a dialysis method, and a combination of these. With the methods, the antibody of the present invention can be readily produced in high yield and high purity.

The antibody or an active fragment thereof of the present invention may be further modified by various molecules such as polyethylene glycol (PEG), radioactive materials, toxins, and the like. As the method for modifying the antibody, known methods in the art can be used.

Furthermore, other proteins may be fused to the N- or C-terminal end of the antibody of the present invention (Clinical Cancer Research, 2004, 10, 1274-1281). The protein to be fused can be appropriately selected by those skilled in the art.

A pharmaceutical composition comprising the antibody or an antibody fragment thereof of the present invention (pharmaceutical composition of the present invention) can be administered systemically or topically, orally or parenterally. Examples of the parenteral administration include intravenous injection, such as infusion, intramuscular injection, epidural injection, intracerebroventricular injection, intraperitoneal injection, subcutaneous injection, intranasal administration, and inhalation.

The pharmaceutical composition of the present invention is useful as a medicament for the treatment and/or prevention of a CB1 receptor-related disease.

Examples of the CB1 receptor-related disease include obesity, Alstrom syndrome, Prader-Willi syndrome, Bardet-Biedl syndrome, Albright hereditary osteodystrophy, symptomatic obesity including SIM1 deletion syndrome, diabetes and its related complications, insulin resistance, type II diabetes, prediabetes, metabolic syndrome, dyslipidemia, nonalcoholic steatohepatitis (NASH), nonalcoholic fatty liver disease (NAFLD), diabetic gastroparesis, gastroparesis, liver disease such as primary biliary cirrhosis, kidney fibrosis, chronic kidney disease, focal segmental glomerulosclerosis (FSGS), diabetic nephropathy, Alport syndrome, hypertensive kidney disease, nephrotic syndrome, steroid resistant nephrotic syndrome, minute change type nephrotic syndrome, membranous nephropathy, idiopathic membranous nephropathy, membranous proliferative glomerulonephritis (MPGN), immunoconjugate-mediated MPGN, complement-mediated MPGN, lupus nephritis, post-infection glomerulonephritis, thin basement membrane disease, mesangial proliferative glomerulonephritis, amyloidosis (primary), clq nephropathy, rapidly-progressive GN, anti-GBM disease, C3 glomerulonephritis, hypertensive nephrosclerosis, IgA nephropathy, proteinuric kidney disease, microalbuminuria, microalbuminuria kidney disease, etc., diabetic neuropathy, focal segmental glomerulosclerosis, osteoporosis, atherosclerosis, inflammatory disease, cardiovascular disease, cancer, pain, systemic sclerosis, multiple sclerosis spasticity, glaucoma and nicotine intoxication, anxiety, depression, transplant-related FSGS, transplant-related nephrotic syndrome, transplant-related proteinuria, cholestatic liver disease, polycystic kidney disease, multiple cystic kidney disease, autosomal dominant polycystic kidney disease (ADPKD), and the like. The pharmaceutical composition of the present invention is very useful particularly as a medicament for the treatment and/or prevention of obesity, diabetes, and NASH.

The antibody or an antibody fragment thereof of the present invention or the pharmaceutical composition of the present invention may be used in combination with other active ingredients or drugs containing the active ingredients and administered as a concomitant agent, for
(1) supplementing and/or enhancing the therapeutic effect of the pharmaceutical composition of the present invention,
(2) improving in kinetics or absorption or reducing in dosage of the pharmaceutical composition of the present invention, and/or
(3) reducing the side effects of the pharmaceutical composition of the present invention.

Examples of the drug containing other active ingredients include CB1 receptor antibodies other than the antibody or an antibody fragment thereof of the present invention or CB1 receptor antagonists such as small molecule CB1 receptor inhibitors. Examples of the small molecule CB1 receptor inhibitor include rimonabant, taranabant, AM251, AM1387, AM4113, cannabigerol, ibipinabant, otenabant, surinabant, tetrahydrocannabivarin, virodhamine, AM6545, and the like.

The concomitant agent of the antibody or an antibody fragment thereof of the present invention in combination with other active ingredients may be administered in the form of a combination drug containing both components in one formulation, or may be administered in separate formulations (the pharmaceutical composition of the present invention and the drug containing other active ingredients). When administered in separate formulations, they can be co-administered or administered with time difference. The administration with time difference may be performed by administering the pharmaceutical composition of the present invention, then the other agent, or by administering the other agent, then the pharmaceutical composition of the present invention, in which each administration method may be the same or different.

The effective dosage of the pharmaceutical composition according to the present invention is selected from the range of 0.01 mg to 100 mg per kg of body weight per dose. Alternatively, it can be selected from a dosage of 5 to 5000 mg, preferably 10 to 500 mg, per patient. However, the pharmaceutical composition of the present invention is not limited to these doses. In addition, the administration period can be appropriately selected according to the age and symptom of the patient, and the dosage can be scaled using methods well known in the art (for example, Mordenti et al. (1991) Pharmaceut. Res. 8: 1351-1359). The pharmaceutical composition of the present invention may further comprise a pharmaceutically acceptable carrier or additive, depending on the route of administration. Examples of the carrier and additive include water, a pharmaceutically acceptable organic solvent, collagen, polyvinyl alcohol, polyvinylpyrrolidone, sodium alginate, watersoluble dextran, pectin, methylcellulose, ethylcellulose, casein, diglycerin, propylene glycol, polyethylene glycol, petrolatum, human serum albumin (HSA), mannitol, sorbitol, lactose, and a surfactant acceptable as a pharmaceutical additive. The additives used are selected as appropriate or in combination from the above, depending on the dosage form, but are not limited thereto.

The present invention encompasses a polynucleotide encoding a heavy chain variable region and/or a light chain variable region of the antibody of the present invention. The polynucleotide encoding the heavy chain variable region of the antibody of the present invention may further encode the heavy chain constant region. The polynucleotide encoding the light chain variable region of the antibody of the present invention may further encode the light chain constant region. Also, the present invention further encompasses an expression vector comprising at least one of the polynucleotides.

The polynucleotide is not particularly limited as long as it encodes a light chain variable region or a heavy chain variable region of the antibody of the present invention, and it is a polymer consisting of nucleotides such as a plurality of deoxyribonucleic acids (DNA) or ribonucleic acids (RNA). It may contain a non-natural nucleotide. The polynucleotide of the present invention can be used for producing antibodies by genetically engineered methods. The polynucleotide of the present invention can also be used as a probe for screening an antibody having the same function as the antibody of the present invention. That is, a polynucleotide encoding the antibody of the present invention or a portion thereof can be used as a probe in techniques such as hybridization, gene amplification techniques (e.g., PCR) or the like to obtain a DNA that hybridizes with the polynucleotide under stringent conditions and encodes an antibody having equivalent activity to the antibody of the present invention. Such DNA is also included in the polynucleotide of the present invention.

Hybridization techniques (Sambrook, J et al., Molecular Cloning 2nd ed., 9.47-9.58, Cold Spring Harbor Lab. press, 1989) are techniques well known to those skilled in the art. Examples of conditions for hybridization include low stringent conditions. A low stringent condition is, for example, a condition of 42°C, 0.1 × SSC, 0.1% SDS, preferably a condition of 50°C, 0.1 × SSC, 0.1% SDS in washing after hybridization. Examples of more preferred hybridization conditions include high stringent conditions. A high stringent condition is, for example, a condition of 65°C, 5 x SSC and 0.1% SDS. In these conditions, it can be expected that polynucleotides having high homology are efficiently obtained as the temperature increases. However, multiple factors such as temperature and salt concentration are considered to affect the stringency of hybridization. Those skilled in the art can achieve the same stringency by selecting these factors as appropriate.

Antibodies that are functionally equivalent to the antibody of the present invention, encoded by the polynucleotides obtained by these hybridization and gene amplification techniques, typically have high homology with the antibody in amino acid sequence. The antibody of the present invention also includes an antibody that is functionally equivalent to the antibody of the present invention and has high homology with the antibody in amino acid sequence. High homology usually refers to at least 75% or more identity, preferably 85% or more identity, further preferably 95% or more identity in amino acid level. The homology of a polypeptide can be determined according to the algorithm described in the document (Wilbur, W. J. and Lipman, D. J. Proc. Natl. Acad. Sci. USA (1983) 80, 726-730).

The antibody or an antibody fragment thereof of the present invention specifically binds to a CB1 receptor and can thus be used for detecting a CB1 receptor in a biological sample. Examples of the biological sample include blood, plasma, serum, urine, an organ, a tissue, bone marrow, and a lymph node. Accordingly, a kit comprising the antibody of the present invention is available as a kit for detecting CB1 receptor. The kit comprises the antibody or an antibody fragment thereof of the present invention, and may further comprise a secondary antibody for labelling, a substrate necessary for detection of the labelling, a carrier, a wash buffer, a sample dilution, an enzyme substrate, a reaction stop solution, a CB1 receptor protein as a purified standard substance, an instruction for use, and the like.

### [EXAMPLES]

Hereinafter, the present invention will be explained in more detail by way of Examples of the present invention, but the present invention is not limited to.

### Example 1: Production of anti-human CB1 receptor mouse antibody by hybridoma method

A gene encoding ΔN-human CB1 receptor (T210A), a human CB1 receptor mutant in which the full length of a human CB1 receptor (UniProtKB/Swiss-Prot: P21554) or an extracellular N-terminal region (amino acids positions 1 to 116) of the human CB1 receptor was deleted and a T210A mutation was introduced, was used as an antigen and DNA-immunized to CB1 receptor gene knock-out ICR female mice. DNA immunization was repeated two or three times at two-week intervals and boosted by intraperitoneally administering Expi293 cells (Thermo Fisher SCIENTIFIC) transiently expressing the human CB1 receptor one week after final immunization. After three days, spleen was removed, and spleen cells and mouse myeloma cells (p3x6363-Ag8., Tokyo Oncology Institute) were fused by the PEG method, and selection was made in a medium containing hypoxanthine, aminopterin and thymidine.

In the screening of antibodies showing anti-human CB1 receptor-specific binding, clones showing a signal specific to the human CB1 receptor were selected by reacting the hybridoma culture supernatant with Expi293 cells transiently expressing the human CB1 receptor and Expi293 cells transiently expressing the human CB2 receptor, respectively, and detecting with an Alexa488 labeled anti-mouse IgG antibody (Thermo Fisher SCIENTIFIC) using MirrorBall.

The anti-human CB1 receptor inhibitory antibody was selected by cAMP assay using HitHunter (registered trademark) cAMP kit (DiscoverX). CHO cells stably expressing the human CB1 receptor (hCB1 receptor/CHO) were seeded on a 384 well plate at 1 x 10⁴ cells/well, and cultured O/N at 37°C under a 5% CO₂ saturated water vapor pressure, then a culture supernatant of the hybridoma was added, and the mixture was reacted at room temperature for 3 hours. After washing twice with 20 mM Hepes-HBSS (pH7.4), the mixture was reacted at room temperature for 30 minutes so that the final concentration of CP55940 (CB1 receptor agonist) was 10 nM and the final concentration of Forskolin was 10 µM, then cAMP antibody reagent and cAMP working detection solution were added, and the mixture was further reacted while being shielded from light at room temperature for 1 hour. Thereafter, cAMP solution A was added, and the mixture was reacted while being shielded from light at room temperature for 3 hours, and then luminescence was detected. In a control in which only the medium was reacted instead of the hybridoma supernatant, the inhibition rate was calculated by setting the signal value of CP55940+ and Forskolin+ as the inhibition rate of 0% and the signal value of CP55940- and Forskolin+ as the inhibition rate of 100%, and a clone showing an inhibition rate of 50% or more was selected as a human CB1 receptor inhibitory antibody.

### Example 2: Production of an anti-human CB1 receptor mouse antibody by immune phage antibody library

About 1/20 amount (about 1 x 10⁷ cells) of the spleen cells isolated from the mouse immunized with the full length of the human CB1 receptor of Example 1 was subjected to production of an immune phage antibody library. mRNA was extracted from the spleen cells by Trizol and Dynabeads mRNA purification kit (Thermo Fisher SCIENTIFIC), and a cDNA library was synthesized by SuperscriptIII first-strand cDNA synthesis kit (Thermo Fisher SCIENTIFIC). Primer mixes encoding the N-terminal region and the J chain of the mouse antibody germline registered in IMGT (registered trademark) database were designed as a Forward primer set and a Reverse primer set, respectively, and using these, the variable regions of the K chain and the H chain were amplified by KOD plus DNA polymerase (TOYOBO), respectively. These were linked by assembly PCR with a G4Sx3 linker to form a scFv antibody gene library, and inserted into a phagemid vector by restriction enzyme treatment. This was transformed into an E. coli TG1 strain by an electroporation method and O/N cultured on LB agar medium to obtain a scFv library (E. coli stock) with a diversity of about 10⁹. The phage antibody library was prepared by amplifying this E. coli stock in LB liquid medium to the logarithmic growth phase, then infecting with M13KO7 helper phage, inducing into LB liquid medium culture supernatant at 26°C, O/N, and enriching with PEG-NaCl. The phage antibody library was first reacted with Expi293 cells, and the operation of collecting the phage group that did not bind as "phage that does not non-specifically bind to cells" (subtraction) was repeated five times. This supernatant was reacted with Expi293 cells transiently expressing the human CB1 receptor, washed ten times with PBS, and then phage bound to the human CB1 receptor on the cell surface was eluted with 0.1 M glycine-HCl. This was neutralized with 1/10 amount of Tris-HCl (pH8.0), and infected with an E. coli TG1 strain, and the human CB1 receptor binding clone was enriched. The operation of enriching the human CB1 receptor binding clone (panning) was repeated three times. The enriched phage antibody library was infected with TG1, and monocloned in a 96 well plate, and using the scFv protein induced in the culture supernatant, an anti-human CB1 receptor-specific antibody and an anti-human CB1 receptor inhibitory antibody were selected in the same manner as in the method described in Example 1.

### Example 3: Determination of antibody sequences

From the hybridoma cells identified as the human CB1 receptor inhibitory antibody in Example 1 and the phagemid vectors derived from the phage antibody identified as the human CB1 receptor inhibitory antibody in Example 2, the amino acid sequences of the light chain variable region and the heavy chain variable region of the antibody were determined according to a conventional method. The identified sequences are shown in Table 1.

**[Table 1]**

| Antibody | Variable region | SEQ ID No. | | SEQ ID No. | Variable region | SEQ ID No. | | SEQ ID No. |
|---|---|---|---|---|---|---|---|---|
| mIML1-2 | Light chain | 1 | CDR1 | 19 | Heavy chain | 7 | CDR1 | 24 |
| | | | CDR2 | 20 | | | CDR2 | 25 |
| | | | CDR3 | 21 | | | CDR3 | 26 |
| m20C3 | Light chain | 2 | CDR1 | 19 | Heavy chain | 8 | CDR1 | 24 |
| | | | CDR2 | 20 | | | CDR2 | 27 |
| | | | CDR3 | 21 | | | CDR3 | 26 |
| m2F8 | Light chain | 3 | CDR1 | 40 | Heavy chain | 9 | CDR1 | 24 |
| | | | CDR2 | 20 | | | CDR2 | 28 |
| | | | CDR3 | 23 | | | CDR3 | 29 |
| m4D7 | Light chain | 4 | CDR1 | 22 | Heavy chain | 10 | CDR1 | 24 |
| | | | CDR2 | 20 | | | CDR2 | 28 |
| | | | CDR3 | 23 | | | CDR3 | 30 |
| m24C4 | Light chain | 4 | CDR1 | 22 | Heavy chain | 11 | CDR1 | 24 |
| | | | CDR2 | 20 | | | CDR2 | 28 |
| | | | CDR3 | 23 | | | CDR3 | 31 |
| m26C7 | Light chain | 4 | CDR1 | 22 | Heavy chain | 12 | CDR1 | 24 |
| | | | CDR2 | 20 | | | CDR2 | 28 |
| | | | CDR3 | 23 | | | CDR3 | 32 |

### Example 4: Evaluation of neutralizing activity

The established hybridomas were cultured in serum-free medium and the culture supernatant was subjected to affinity purification by Protein G column and gel filtration purification to give a purified antibody. The neutralizing activity of this purified antibody was measured by the following method using cAMP activity and Ca²⁺ influx as indices.

The cAMP activity was evaluated by HitHunter cAMP kit. The hCB1 receptor/CHO described in Example 1 was seeded on a 384 well plate at 7.5 x 10³ cells/well, and cultured O/N at 37°C under a 5% CO₂ saturated water vapor pressure, an antibody diluted with 0.1% BSA/20 mM HEPES-HBSS (pH7.4) was added, and the mixture was reacted at room temperature for 1.5 hours. After washing twice with 20 mM HEPES-HBSS (pH7.4), the mixture was reacted at room temperature for 30 minutes so that the final concentration of CP55940 was 3 nM and the final concentration of Forskolin was 10 µM, then cAMP antibody reagent and a cAMP working detection solution were added, and the mixture was further reacted while being shielded from light at room temperature for 1 hour. cAMP solution A was added, and the mixture was reacted while being shielded from light at room temperature for 3 hours, and then luminescence was detected. In the control without addition of the antibody, the inhibition rate was determined by setting the signal value of CP55940+ and Forskolin+ as the inhibition rate of 0% and the signal value of CP55940- and Forskolin+ as the inhibition rate of 100%, and the antibody concentration showing 50% inhibition was calculated as IC50 (nM) and expressed as Average ± SD (Table 2).

Ca²⁺ influx was evaluated using FLIPR Penta. The antibody diluent diluted with the medium was added to human CB1 receptor-expressing Gα16CHO cells previously incorporating Ca²⁺ indicator, and the mixture was reacted in a 5% CO₂ incubator at 37°C for 60 minutes. After washing with 20 mM HEPES-HBSS (pH7.4) twice, Ca²⁺ influx by addition of CP55940 having a final concentration of 10 µM was measured. The inhibition rate was calculated by setting the signal when CP55940 was not added as the inhibition rate of 100% and the signal when CP55940 was added and the antibody was not added as the inhibition rate of 0%, and the antibody concentration showing the inhibition rate of 50% was taken as IC50. The evaluation was performed at least three times, and IC50 was expressed as Average ± SD (Table 2).

As a result, in either evaluation system, it had much stronger human CB1 receptor inhibitory activity than rimonabant, which is a small molecule inhibitor used as a positive control, and exhibited an inverse agonist-like action.

**[Table 2]**

| Antibody | cAMP assay IC50 (nM) | Ca²⁺ influx assay IC50 (nM) |
|---|---|---|
| mIML1-2 | 5.9 ± 2.0 | 1.77 ± 0.96 |
| m2F8 | 4.9 ± 1.4 | 0.90 ± 0.58 |
| m4D7 | 3.9 ± 0.6 | 1.29 ± 0.58 |
| m24C4 | 5.0 ± 1.0 | 1.22 ± 0.63 |
| m26C7 | 6.5 ± 2.7 | 1.85 ± 0.80 |
| Rimonabant | 15.8 ± 6.6 | 3629 ± 1147 |

### Example 5: Humanization of antibody

Humanization was performed for mIML1-2, m2F8 by the methods described below.

Using the antibody sequence analysis software abYsis, numbering by Kabat definition (Kabat numbering (Wu, T.T. and Kabat, E.A., J Exp. Med. Aug 1; 132(2): 211-50. (1970)) and CDR specification were performed. Next, human reproductive system acceptor sequences that are highly homologous to the variable region sequences of the heavy chain and the light chain of the amino acid sequence of the mouse antibody were searched with sequence analysis software AbYsis. Humanization was performed by transplanting mouse antibody heavy chains a CDR1, a CDR2, and a CDR3 and mouse antibody light chains a CDR1, a CDR2, and a CDR3, respectively to this human framework sequence. For the J-chain region, sequences that are highly homologous to J-chain region sequences of the mouse antibody were searched and selected with IMGT (http://www.imgt.org/). As a result, humanized monoclonal antibodies (hIML1-2, hIML1-2-2, h2F8) showing a human CB1 receptor inhibitory activity almost equivalent to that of the mouse antibody were obtained. In addition, for hIML1-2-2, mutants having single mutation introduced into the framework and CDRs were comprehensively produced, and mutations that improve the affinity for the human CB1 receptor were identified. Next, these mutations were superimposed to obtain a humanized monoclonal antibody (hIML1-2-OP1) having improved human CB1 receptor inhibitory activity. These sequences are shown in Table 3.

**[Table 3]**

| Antibody | Variable region | SEQ ID No. | | SEQ ID No. | Variable region | SEQ ID No. | | SEQ ID No. |
|---|---|---|---|---|---|---|---|---|
| hIML1-2 | Light chain | 13 | CDR1 | 19 | Heavy chain | 16 | CDR1 | 24 |
| | | | CDR2 | 20 | | | CDR2 | 27 |
| | | | CDR3 | 21 | | | CDR3 | 26 |
| hIML1-2 -2 | Light chain | 13 | CDR1 | 19 | Heavy chain | 52 | CDR1 | 24 |
| | | | CDR2 | 20 | | | CDR2 | 25 |
| | | | CDR3 | 21 | | | CDR3 | 26 |
| hIML1-2 - OP1 | Light chain | 14 | CDR1 | 33 | Heavy chain | 17 | CDR1 | 24 |
| | | | CDR2 | 34 | | | CDR2 | 36 |
| | | | CDR3 | 35 | | | CDR3 | 26 |
| h2F8 | Light chain | 15 | CDR1 | 40 | Heavy chain | 18 | CDR1 | 24 |
| | | | CDR2 | 20 | | | CDR2 | 28 |
| | | | CDR3 | 23 | | | CDR3 | 29 |

In addition, the human CB1 receptor inhibitory activity was evaluated using cAMP activity and Ca²⁺ influx as indices in the same manner as in Example 4. For comparison with the prior antibodies, Nimacimab (BIRD ROCK BIO) and M5 and M7 described in Patent Document 5 (WO2019/211665) were prepared by a method using ExpiCHO cells. As a result of comparative evaluation of the human CB1 receptor inhibitory activity by cAMP activity at a final concentration of CP55940 of 10 nM and at a final concentration of Forskolin of 10 µM and Ca²⁺ influx at a final concentration of CP55940 of 10 µM, it was shown that the antibodies described in the present invention exhibit a very strong human CB1 receptor inhibitory activity that exceeds several times to several tens of times as shown in Table 4, and have an inhibitory activity superior to that of the prior antibodies.

**[Table 4]**

| Antibody | cAMP assay IC50 (nM) | Ca²⁺ influx assay IC50 (nM) |
|---|---|---|
| hIML1-2-OP1 | 8.5 ± 2.4 | 0.55 ± 0.24 |
| h2F8 | 5.9 ± 2.9 | 2.03 ± 0.68 |
| Nimacimab | 28.6 ± 10.0 | 2.62 ± 0.92 |
| M5 | 117.0 ± 40.6 | 4.02 ± 2.27 |
| M7 | 23.0 ± 22.4 | 21.69 ± 9.61 |
| Rimonabant | 53.4 ± 14.2 | 3629 ± 1147 |

### Example 6: Single dose POM study in human CB1 receptor knock-in mice (hereinafter, hCB1 receptor-KI (KI/KI) mice) (Evaluation of small intestine transport capacity)

### (1) Production of hCB1 receptor-KI (KI/KI) mice

The mouse CB1 receptor gene (Gene ID: 12801) is composed of multiple exons, and the final exon contains the full length of ORF (open reading frame). The full-length ORF (CCDS ID: 18025.1) of the mouse CB1 receptor gene was removed while the full-length ORF (CCDS ID: 5015.1) of the human CB1 receptor gene to which a DYKDDDDK (SEQ ID NO: 53) tag was added at the C-terminal was inserted, thereby hCB1 receptor-KI (KI/KI) mice in which the CB1 receptor protein expressed was fully humanized were prepared.

DNA fragments to be used as homologous recombination arms were obtained by PCR amplification of mouse genomic sequences about 2 kb (kilobase) upstream and downstream of the ORF of the mouse CB1 receptor gene, respectively. The homologous recombination arms were designed so that only ORF was removed. The homologous recombination arms were seamlessly connected to both sides of the full-length sequence of ORF of the human CB1 receptor gene to which a DYKDDDDK (SEQ ID NO: 53) tag was added at the C-terminal, respectively, to produce a targeting vector. The targeting vector was subjected to homologous recombination to produce a hCB1 receptor-KI (KI/+) C57/BL6 mouse. The produced hCB1 receptor-KI (KI/+) C57/BL6 mouse was confirmed that the full-length ORF of the mouse CB1 receptor gene was correctly replaced to the full-length ORF of the human CB1 receptor gene and there were no extra insertions or deletions of sequences by PCR and DNA nucleotide sequence analysis. The hCB1 receptor-KI (KI/+) mice were crossed to produce a hCB1 receptor-KI (KI/KI) C57/BL6 mouse.

### (2) Single dose hCB1 receptor POM study in hCB1 receptor-KI (KI/KI) mice

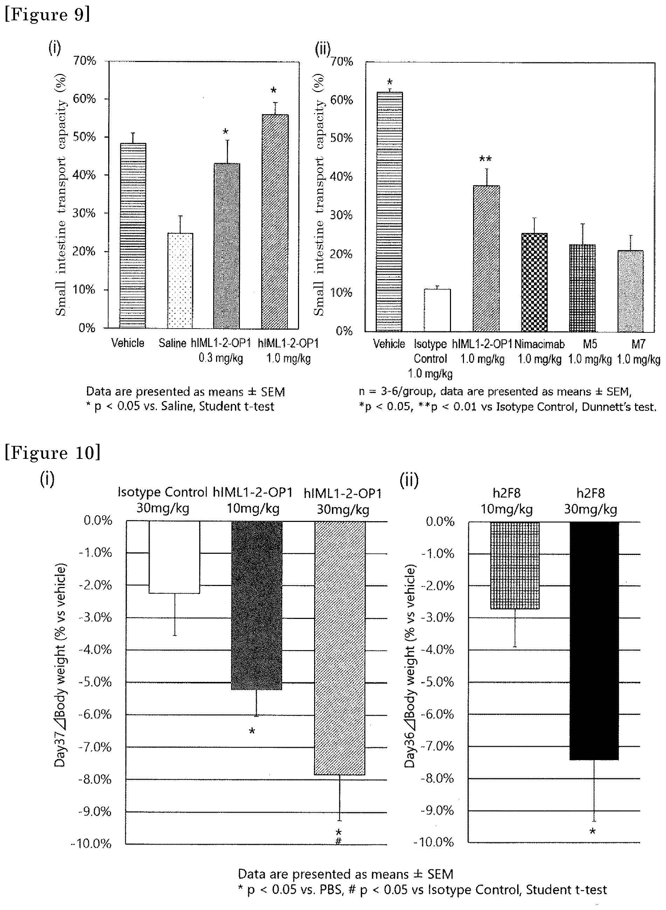

To 13-week-old hCB1 receptor KI (KI/KI) mice, hIML1-2-OP1 (0.3 mg/kg, 1 mg/kg) which is an anti-human CB1 receptor humanized antibody shown in Example 5 or Saline was subcutaneously administered once, and the mice were fasted. The following day, the CB1 receptor agonist CP55940 (0.05 mg/kg) was administered intraperitoneally, and 30 minutes later, 10% carbon suspension was orally administered at 0.1 mL/mouse. The small intestine was taken out 30 minutes after the administration of the carbon suspension, and the small intestine transport capacity was evaluated using the moving distance of the carbon suspension as an index. As a result, a significant decrease in small intestine transport capacity by administration of CP55940 was confirmed, and hIML1-2-OP1 showed significant inhibitory activity at both 0.3 mg/kg and 1.0 mg/kg against the decrease (Figure 9-i). In the same manner, an isotype control antibody (1 mg/kg), Nimacimab (BIRD ROCK BIO) (1 mg/kg) known as a prior antibody, and M5 (1 mg/kg) and M7 (1 mg/kg) described in Patent Document 5 (WO2019/211665) were also evaluated in comparison with hIML1-2-OP1 (1 mg/kg). As a result, while hIML1-2-OP1 reproduced a strong inhibitory effect, the prior antibodies, Nimacimab, M5 and M7, did not show significant inhibition even at 1 mg/kg, and it was shown that the antibody described in the present invention has excellent inhibitory activity against known antibodies also in vivo (Figure 9-ii).

### Example 7: Anti-obesity effect in hCB1 receptor-KI (KI/KI) mouse DIO model

Seven-week-old hCB1 receptor KI (KI/KI) mice were loaded with a 60% high-fat diet (58Y1) and assigned to a PBS-administered group and an antibody-administered group based on body weight at 5 weeks from the start of loading. Thereafter, hIML1-2-OP1, which is an anti-human CB1 receptor humanized antibody prepared in Example 5, and an Isotype Control antibody were repeatedly administered by subcutaneous administration once a week at 10 mg/kg or 30 mg/kg for a total of six times. Body weight and food intake were monitored once a week to evaluate the anti-obesity effect. In addition, about 6 weeks after the administration (Day 44), dissection was performed, and the fatty liver improving action was evaluated by quantifying the amount of triglyceride in the liver. In addition, h2F8 was also subcutaneously administered five times in total in the same manner, and the anti-obesity effect and the fatty liver improving effect were evaluated using about 5 weeks after the administration (Day 36) as an endpoint.

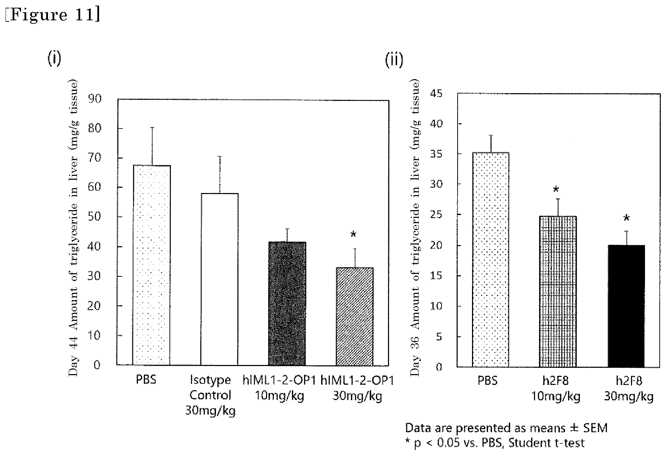

As a result, in the hIML1-2-OP1 and h2F8 administration groups, an inhibitory effect on the body weight gain due to the high-fat diet load was observed, and a dose-dependent anti-obesity effect was confirmed. The weight loss effect on the PBS group about 5 weeks after the start of antibody administration was -5.0% and -7.6% in the 10 mg/kg administration group and the 30 mg/kg administration group of hIML1-2-OP1, respectively, and both were significant effects (Figure 10-i). Also for h2F8, the weight loss effect was -7.4% in the 30 mg/kg administration group, showing a significant effect (Figure 10-ii). On the other hand, regarding the amount of food intake during the antibody administration period, no significant change was observed with respect to the PBS administration group, and an anti-obesity effect was observed without suppressing food intake. In addition, as a result of quantifying the amount of triglyceride in the liver on Day 44 (hIML1-2-OP1) and Day 36 (h2F8) set as the endpoints, a 38% and 51% was observed in the 10 mg/kg administration group and the 30 mg/kg administration group of hIML1-2-OP1, respectively, as compared with the PBS group (Figure 11-i), and a 30% and 43% decrease was observed in the 10 mg/kg administration group and the 30 mg/kg administration group of h2F8, respectively, as compared with the PBS group (Figure 11-ii), showing a strong reducing effect of the amount of fat in the liver.

### [INDUSTRIAL APPLICABILITY]

The antibody or an antibody fragment of the present invention can be used for detecting a CB1 receptor in a biological sample. Furthermore, the pharmaceutical composition comprising the antibody or an antibody fragment of the present invention is very useful as a medicament for the treatment or prevention of CB1 receptor-related diseases.

## Claims

1. An antibody or an antibody fragment thereof that binds to a human cannabinoid type 1 (CB1) receptor, comprising:
a light chain variable region including
a CDR1 having the amino acid sequence of SEQ ID NO: 44,
a CDR2 having the amino acid sequence of SEQ ID NO: 45, and
a CDR3 having the amino acid sequence of SEQ ID NO: 46, and
a heavy chain variable region including
a CDR1 having the amino acid sequence of SEQ ID NO: 24,
a CDR2 having the amino acid sequence of SEQ ID NO: 47, and
a CDR3 having the amino acid sequence of SEQ ID NO: 26.

2. An antibody or an antibody fragment thereof that binds to a human cannabinoid type 1 (CB1) receptor, comprising:
a1) a light chain variable region including
a CDR1 having the amino acid sequence of SEQ ID NO: 33,
a CDR2 having the amino acid sequence of SEQ ID NO: 34, and
a CDR3 having the amino acid sequence of SEQ ID NO: 35, and
a2) a heavy chain variable region including
a CDR1 having the amino acid sequence of SEQ ID NO: 24,
a CDR2 having the amino acid sequence of SEQ ID NO: 36, and
a CDR3 having the amino acid sequence of SEQ ID NO: 26;
b1) a light chain variable region including
a CDR1 having the amino acid sequence of SEQ ID NO: 40,
a CDR2 having the amino acid sequence of SEQ ID NO: 20, and
a CDR3 having the amino acid sequence of SEQ ID NO: 23, and
b2) a heavy chain variable region including
a CDR1 having the amino acid sequence of SEQ ID NO: 24,
a CDR2 having the amino acid sequence of SEQ ID NO: 28, and
a CDR3 having the amino acid sequence of SEQ ID NO: 29;
c1) a light chain variable region including
a CDR1 having the amino acid sequence of SEQ ID NO: 19,
a CDR2 having the amino acid sequence of SEQ ID NO: 20, and
a CDR3 having the amino acid sequence of SEQ ID NO: 21, and
a heavy chain variable region including
c2-1) a CDR1 having the amino acid sequence of SEQ ID NO: 24,
a CDR2 having the amino acid sequence of SEQ ID NO: 27, and
a CDR3 having the amino acid sequence of SEQ ID NO: 26, or
c2-2) a CDR1 having the amino acid sequence of SEQ ID NO: 24,
a CDR2 having the amino acid sequence of SEQ ID NO: 25, and
a CDR3 having the amino acid sequence of SEQ ID NO: 26; or
d1) a light chain variable region including
a CDR1 having the amino acid sequence of SEQ ID NO: 22,
a CDR2 having the amino acid sequence of SEQ ID NO: 20, and
a CDR3 having the amino acid sequence of SEQ ID NO: 23, and
a heavy chain variable region including
d2-1) a CDR1 having the amino acid sequence of SEQ ID NO: 24,
a CDR2 having the amino acid sequence of SEQ ID NO: 28, and
a CDR3 having the amino acid sequence of SEQ ID NO: 30,
d2-2) a CDR1 having the amino acid sequence of SEQ ID NO: 24,
a CDR2 having the amino acid sequence of SEQ ID NO: 28, and
a CDR3 having the amino acid sequence of SEQ ID NO: 31, or
d2-3) a CDR1 having the amino acid sequence of SEQ ID NO: 24,
a CDR2 having the amino acid sequence of SEQ ID NO: 28, and
a CDR3 having the amino acid sequence of SEQ ID NO: 32.

3. The antibody or an antibody fragment thereof according to claim 1 or 2, comprising:
a light chain variable region having
the amino acid sequences of SEQ ID NOs: 13 to 15, or
the amino acid sequences at least 95% identical to the amino acid sequences of SEQ ID NOs: 13 to 15, and
a heavy chain variable region having
the amino acid sequences of SEQ ID NOs: 16 to 18, and 52, or
the amino acid sequences at least 95% identical to the amino acid sequences of SEQ ID NOs: 16 to 18, and 52.

4. The antibody or an antibody fragment thereof according to any one of claims 1 to 3, comprising:
a1) a light chain variable region having the amino acid sequence of SEQ ID NO: 14, and
a heavy chain variable region having the amino acid sequence of SEQ ID NO: 17;
a2) a light chain variable region having the amino acid sequence of SEQ ID NO: 15, and
a heavy chain variable region having the amino acid sequence of SEQ ID NO: 18;
a3) a light chain variable region having the amino acid sequence of SEQ ID NO: 13, and
a heavy chain variable region having the amino acid sequence of SEQ ID NO: 16; or
a4) a light chain variable region having the amino acid sequence of SEQ ID NO: 13, and
a heavy chain variable region having the amino acid sequence of SEQ ID NO: 52.

5. The antibody or an antibody fragment thereof according to any one of claims 1 to 4, further comprising:
a heavy chain constant region having the amino acid sequence of SEQ ID NO: 37, and
a light chain constant region having the amino acid sequence of SEQ ID NO: 38.

6. A pharmaceutical composition comprising the antibody or an antibody fragment thereof according to any one of claims 1 to 5.

7. The pharmaceutical composition according to claim 6, which is a therapeutic and/or preventive agent for obesity, diabetes and/or nonalcoholic steatohepatitis (NASH).

8. A polynucleotide encoding the heavy chain variable region of the antibody according to claim 3 or 4, and
further optionally encoding the heavy chain constant region of the antibody according to claim 5.

9. A polynucleotide encoding the light chain variable region of the antibody according to claim 3 or 4, and
further optionally encoding the light chain constant region of the antibody according to claim 5.

10. An expression vector comprising the polynucleotide according to claim 8 or 9.
